# EUROPÄISCHE PATENTSCHRIFT

(11) **EP 2 422 731 B1**
(45) Veröffentlichungstag und Bekanntmachung des Hinweises auf die Patenterteilung: **01.11.2017**
(21) Anmeldenummer: 11178495.5
(22) Anmeldetag: 23.08.2011
(51) Int. Cl.: A61B 17/70, A61B 17/00, A61B 90/00

(54) **Aktuator zur Skoliosekorrektur**
Actuator for scoliosis correction
Actionneur pour la correction de la scoliose

(30) Priorität: 26.08.2010 DE 102010047738; 26.08.2010 DE 102010035570
(43) Veröffentlichungstag der Anmeldung: 29.02.2012
(73) Patentinhaber: WITTENSTEIN SE, 97999 Igersheim (DE)
(72) Erfinder: Stauch, Roman, 97959 Assamstadt (DE)
(74) Vertreter: Zimmermann & Partner Patentanwälte mbB

(56) Entgegenhaltungen:
- WO-A1-01/78614
- WO-A2-2006/090380
- DE-A1- 2 417 233
- DE-A1- 2 845 647
- DE-U1-202004 007 242
- FR-A1- 2 843 538
- US-A1- 2006 195 087
- US-A1- 2009 275 984
- US-A1- 2010 094 306
- US-A1- 2010 121 323

## Beschreibung

Die Erfindung betrifft eine Skoliosebehandlungsvorrichtung zur Behandlung von Skoliose beim Menschen nach dem Oberbegriff des Anspruchs 1.

### Stand der Technik

Implantate zur Behandlung von Skoliose sind aus dem Stand der Technik bekannt. Skoliose ist eine Erkrankung der Wirbelsäule, wobei starke Deformationen der Wirbelsäule auftreten. Diese Deformationen werden im Allgemeinen mechanisch behandelt, wobei grundsätzlich zwischen externen Behandlungsmethoden mit einem Korsett einerseits und Behandlungsmethoden mit Hilfe von Implantaten andererseits unterschieden wird. Die Behandlung ist in jedem Fall komplex und langwierig, da die Wirbelsäule nur über einen langen Zeitraum in ihrer Geometrie verändert werden kann. So wird beispielsweise bei einer Behandlung mit einem Korsett davon ausgegangen, dass bei einem Tragen des Korsetts über bis zu 23 Stunden pro Tag etwa zwei bis fünf Jahre eingeplant werden müssen, bis eine Skoliosebehandlung mit dem Korsett abgeschlossen ist.

Behandlungen mit Implantaten weisen den Vorteil auf, dass das Tragen des Korsetts entfällt, sodass die Behandlung zumindest zwischen den Operationen als angenehmer empfunden wird. Bei einer Behandlung mittels Implantaten ist es bekannt, mehrere Wirbelkörper durch zwei Stäbe zu fusionieren oder zu versteifen. Problematisch ist allerdings, dass die Wirbelkörper durch Verknöcherung oder ähnliche Prozesse versteifen, sodass es unter Umständen bis zu einem kompletten Verlust der Mobilität oder einer Verhinderung von weiterem Wachstum kommen kann.

Moderne Systeme versuchen, eine Aufrichtung der Wirbelsäule schrittweise zu erreichen, wobei ein implantiertes Stabsystem im Laufe der Zeit an die sich verändernde Wirbelsäule immer wieder angepasst wird. Nachteilig an dieser Vorgehensweise ist, dass ein Nachstellen des Implantats regelmäßig notwendig wird. Dies bedingt operative Eingriffe unter Vollnarkose, welche insbesondere durch den kurzen zeitlichen Abstand zwischen den Eingriffen eine deutliche psychische Belastung für den Patienten darstellen können.

Alle bisher bekannten Korrekturverfahren weisen den Nachteil auf, dass jede Korrektur in einer Sitzung durchgeführt werden muss und der Korrekturbereich dadurch stark eingeschränkt ist. Grund für die Einschränkung ist der langsame biologische Adaptionsprozess.

Systeme, welche einen implantierbaren Antrieb umfassen, wie beispielsweise in der EP 1 135 076 B1 offenbart, vermeiden häufige Operationen. Alle bisher vorgeschlagenen Antriebe, welche implantierbar ausgeführt sind, haben sich jedoch aus unterschiedlichen Gründen in der Praxis nur wenig durchgesetzt. Insbesondere ist es bisher problematisch, eine ausreichende Kraftwirkung bei ausreichendem Hub des Implantats zu erreichen.

Aus der DE 24 17 233 A1 und der WO 01/78614 A1 sind Distraktionsgeräte für die Verlängerungsosteotomie bekannt, welche zum Verbau in langen Röhrenknochen vorgesehen sind. Aus der US 2006/0195087 A1 und der FR 2 843 538 sind jeweils Geräte für die Skoliosebehandlung bekannt, welche jedoch nicht über einen internen Motor verfügen. Die US 2009/0275984 A1 offenbart ein Gerät zur Skoliosebehandlung mit einem innerhalb eines Schaftelementes angeordneten Motor. Aus der US 2010/0094306 A1 ist ein Gerät für die Rückendistraktion bekannt, wobei ein Antrieb über von außerhalb des Körpers angetriebene Magneten erfolgt. Die DE 28 45 647 A1 offenbart ein Korrekturgerät für die operative Skoliosebehandlung, wobei ein Schaft gedreht wird, um einen Draht aufzuwickeln, welcher wiederum an einem Wirbel befestigt ist, sodass eine Wirbelsäule bei Skoliosebehandlung nach Medial gezogen wird.

### Offenbarung der Erfindung

Aufgabe der Erfindung ist es, die aus dem Stand der Technik bekannten Vorrichtungen zu verbessern. Insbesondere ist es Aufgabe der Erfindung, eine Vorrichtung zur relativen Verschiebung von Knochen, insbesondere ein Implantat zur Behandlung von Skoliose beim Menschen anzugeben, die eine schrittweise Anpassung unter Verringerung oder Reduzierung der operativen Eingriffe mit einem ausreichenden Hub ermöglichen.

Die Aufgabe wird mit einer Vorrichtung nach Anspruch 1 gelöst. Vorteilhafte Weiterbildungen sind den Unteransprüchen zu entnehmen. Ein Aspekt der Erfindung betrifft ein System zur Skoliosekorrektur mit einer Vorrichtung nach Anspruch 1, wobei ein Steuergerät vorgesehen ist, welches dazu eingerichtet ist, die Vorrichtung drahtlos zu steuern oder induktiv mit Energie zu versorgen. Insbesondere ist die Vorrichtung zur operativen Stabilisierung und Korrektur von Skoliose und Deformationen des Brustkorbes geeignet.

Soweit in dieser Anmeldung das Wort "oder" verwendet wird, ist ohne anderslautende, entgegenstehende Aussagen oder Ausdrücke ein nichtausschließendes "oder" im Sinne von "oder auch" gemeint.

Vorteile der Erfindung sind beispielsweise eine feine Verstellmöglichkeit in kleinen Schritten. Dies führt zu geringeren Distraktions- und Kompressionskräften sowie einem größeren Korrekturbereich durch mögliche Nachführung oder einer Kompensation von Skoliosen in der Wachstumsphase.

Vorteilhafterweise umfasst die Vorrichtung einen Antrieb, insbesondere einen Elektromotor. Ein Elektromotor bietet den Vorteil einer hohen Leistungsdichte. Vorzugsweise ist die Achse des Elektromotors außerhalb der beiden Schaftelemente angeordnet. Weiterhin ist die Achse vorzugsweise im Wesentlichen parallel zu einer Achse der Schaftelemente. Dies bedeutet insbesondere, dass die Achse zumindest im Wesentlichen parallel ist zu einer Krümmungsebene der Schaftelemente, falls diese gekrümmt ausgeführt sind. Dabei bedeutet der Ausdruck "längsseits benachbart" von zumindest einem der Schaftelemente, dass der Elektromotor nicht in Längsrichtung oder Verschiebungsrichtung der Schaftelemente genau vor oder hinter beiden Schaftelementen angeordnet ist. Durch die Anordnung des Elektromotors neben den Schaftelementen bzw. neben einer Verschiebungsrichtung der Schaftelemente wird erreicht, dass der Elektromotor nicht die Baulänge der Vorrichtung unnötig verlängert. Auf diese Weise wird ein vorteilhafteres Verhältnis zwischen möglicher Hublänge und Ausgangslänge der gesamten Vorrichtung geschaffen. Daher ist bei besonders bevorzugten Ausführungsformen der Erfindung der Elektromotor bei gekrümmten Schaftelementen zumindest im wesentlichen senkrecht bezüglich der Radialebene der Krümmung seitlich benachbart zu den Schaftelementen angeordnet. Dies bietet den Vorteil, dass eine Implantation im menschlichen Körper einfacher ist, da in diesem Bereich mehr Raum zur Verfügung steht. Weiterhin bietet dies den Vorteil, dass bei einem Antrieb über ein Schneckenrad mit einer Zahnstange der Eingriff zwischen Schneckenrad und Zahnstange auf einem zumindest im wesentlichen ebenen Bereich der Zahnstange vollzogen wird. Dies erleichtert die Kinematik. Allgemein bedeutet der Ausdruck "zumindest im wesentlichen eben" im Zusammenhang mit der Zahnstange, dass die Oberfläche zumindest im wesentlichen ungekrümmt ist, wobei die Zähne für den Antriebseingriff nicht berücksichtigt werden. "Im wesentlichen eben" bedeutet, dass die Krümmung auf der Seite des Eingriffs mit dem Schneckenrad vergleichsweise gering ist, typischerweise höchstens 10% oder höchstens 20% der Krümmung des Schaftelements in Krümmungsebene. Bei weiteren typischen Ausführungsformen ist die Oberfläche der Zahnstange gekrümmt, jedoch weniger stark gekrümmt als die Zahnstange in die andere Richtung entlang ihrer Längsachse gekrümmt ist. Die Zahnstange ist bei diesen Ausführungsformen in zwei Ebenen gekrümmt. Vorzugsweise ist der Elektromotor implantierbar. Dies bedeutet, dass er für einen dauerhaften Verbleib im menschlichen Körper geeignet ist, beispielsweise durch ein geeignetes Gehäuse, welches vorteilhafterweise den Elektromotor verkapselt.

Bei einem weiteren Aspekt der Erfindung wird als Antrieb ein Aktuator aus Formgedächtnislegierung oder einem Piezoelement verwendet. Dieser wird analog zu dem Elektromotor seitlich neben den Schaftelementen angeordnet. Die Ausführungen, welche zu dem Elektromotor in dieser Anmeldung gemacht wurden, insbesondere zur Anordnung, gelten für den Aktuator analog. Ebenso sind auch bevorzugte Merkmale, welche anschließend für eine Vorrichtung mit Elektromotor beschrieben werden, bei einer Vorrichtung mit Aktuator aus einer Formgedächtnislegierung oder einem Piezoelement vorteilhaft einsetzbar. Formgedächtnislegierungen bieten den Vorteil, dass die auf kleinem Raum einsetzbar sind und vorteilhafterweise vollständig biokompatibel sind. Piezoelemente bieten den Vorteil, dass sie sehr klein sind und wenig Raum beanspruchen.

Die Lage des Elektromotors ist vorzugsweise längsseits benachbart neben den Schaftelementen, wobei dies bei gekrümmten Schaftelementen vorzugsweise bedeutet, dass die Achse des Elektromotors zu einer Krümmungsebene der Schaftelemente parallel und lotrecht zu dieser verschoben ist bis außerhalb der Schaftelemente. Der Elektromotor umfasst vorzugsweise einen Stator und einen Rotor, die jeweils mit Elektro- oder Permanent-Magneten bestückt sind.

Die Schaftelemente sind miteinander verbunden und relativ zueinander verschiebbar. Vorzugsweise sind die Schaftelemente teleskopartig ineinander verschiebbar. Dies bietet den Vorteil, dass eine besonders steife Verbindung der Schaftelemente bei gleichzeitiger Verschiebbarkeit geschaffen wird. Die Schaftelemente können unterschiedliche Profilformen aufweisen, beispielsweise haben Hohlprofile den Vorteil, dass sie besonders steif bei geringem Gewicht- und Materialaufwand sind. Eine weitere Möglichkeit ist, ein T-Profil für eines der Schaftelemente zu verwenden, wobei das T-Profil in einem C-Profil, welches das andere Schaftelement bildet, verschoben werden kann. Besonders bevorzugt werden rohrförmige Schaftelemente, wobei der Querschnitt der Rohre vorzugsweise einem Rechteck oder Quadrat, jeweils vorteilhafterweise abgerundet, entspricht. Bezüglich der Geometrie des Querschnitts der Schaftelemente sind auch andere Krümmungen möglich.

Vorzugsweise ist in der Wirkverbindung zwischen dem Elektromotor und dem zweiten Schaftelement ein Getriebe, vorzugsweise mit einer Übersetzung angeordnet. So weist das Getriebe vorzugsweise eine Übersetzung von mindestens 1:250, bevorzugter von mindestens 1:1000, bevorzugter von mindestens 1:2000, und noch bevorzugter von mindestens 1:4000 auf. Das Getriebe ermöglicht die Verwendung eines vergleichsweise kleinen Motors mit einem geringen Drehmoment, um dennoch eine ausweichende Kraft zwischen den Normbefestigungen der Schaftelemente zu erreichen. Dies beeinflusst die Implantierbarkeit stark, da nur ein kleiner Motor verwendet werden muss, dessen Energieversorgung auch induktiv elektromagnetisch erfolgen kann.

Vorzugsweise ist das Getriebe als ein Planetengetriebe ausgebildet oder umfasst ein Planetengetriebe. Planetengetriebe bieten den Vorteil einer besonders großen Übersetzung auf kleinem Raum. Weiterhin weist das Planetengetriebe vorzugsweise einen Durchmesser von weniger als 20 mm, bevorzugter weniger als 15 mm oder weniger als 12 mm auf. Ebenso weist ein Motor vorzugsweise einen Durchmesser von höchstens 20 mm, bevorzugt höchstens 15 mm, noch bevorzugter höchstens 12 mm auf. Motor und Getriebe sind vorzugsweise axial hintereinander angeordnet, sodass sie eine Einheit bilden. Dies bietet den Vorteil eines kompakten Aufbaus. Der Elektromotor erreicht im Zusammenspiel mit dem Getriebe einschließlich einer Kraftübertragung auf die Zahnstange eine Kraftwirkung von mindestens 100 N, bevorzugter mindestens 300 N zwischen den Schaftelementen. Eine solche Kraft ist ausreichend, um eine Skoliosebehandlung zu ermöglichen.

Vorzugsweise ist es möglich, die Schaftelemente um mindestens 40 %, bevorzugter mindestens 50 %, noch bevorzugter mindestens 80% ihrer anfänglichen Länge zu verlängern. Die Länge wird typischerweise zwischen zwei Knochenbefestigungen gemessen. Die Knochenbefestigungen sind jeweils an den Schaftelementen befestigt und ermöglichen vorzugsweise eine Verbindung der Schaftelemente jeweils mit einem Wirbel oder einem Rippenbogen. Vorteilhafterweise sind die Schaftelemente um wenigstens 30 mm, bevorzugter wenigstens 40 mm, relativ zueinander verschiebbar. Dies ermöglicht eine länger andauernde Behandlung, ohne dass ein invasiver Eingriff beim Patienten zwischenzeitlich notwendig wird.

Vorzugsweise ist der Elektromotor in einem Gehäuse angeordnet, welches seitlich auf den ersten Schaft aufgeschoben ist. Das Gehäuse ist vorteilhafterweise mit zumindest einer, vorzugsweise mindestens zwei Schrauben an dem ersten Schaft fixiert. Dabei bedeutet wiederum seitlich, dass das Gehäuse in axialer Richtung bezüglich der Krümmung aufgeschoben ist, also in einer Richtung zumindest im Wesentlichen senkrecht zu der Krümmungsebene. Dies bietet den Vorteil, dass der Elektromotor ggf. mit dem Getriebe im Gehäuse integriert als modulares Element ausgetauscht werden kann. Das seitliche Aufschieben bietet den Vorteil, dass seitlich der Schaftelemente im menschlichen Körper Platz für einen solchen Antrieb ist. Vorzugsweise ist der Elektromotor zusammen mit dem Getriebe und gegebenenfalls einem Antriebszahnrad, wie beispielsweise einem Ritzel, einem Gewinde oder einer Spindel, in dem Gehäuse angeordnet, welches seitlich auf den ersten Schaft aufgeschoben ist. Dies bietet den Vorteil eines kompakten Aufbaus. Bei typischen Ausführungsformen ist das Gehäuse integral mit dem ersten Schaftelement ausgeführt. Der Motor ist in diesem Fall vorzugsweise längsseitig benachbart zum zweiten Schaftelement oder zu einer Verfahrrichtung des zweiten Schaftelements angeordnet. Dabei bedeutet "integral" insbesondere, dass das Gehäuse fest mit dem ersten Schaft verbunden ist oder in diesen integriert ist.

Vorzugsweise wird als Elektromotor ein Gleichstrommotor oder ein Schrittmotor verwendet. Gleichstrommotoren bieten den Vorteil einer hohen Leistungsdichte und eines ruhigen Laufs. Schrittmotoren bieten den Vorteil einer exakten Einstellmöglichkeit der Verschiebung der Schaftelemente relativ zueinander. Vorteilhafte Ausführungsformen der Erfindung sind ungebremst. Der Ausdruck "ungebremst" bedeutet, dass die Vorrichtung keine Bremse oder Raste aufweist, wobei zu berücksichtigen ist, dass insbesondere mit Hilfe eines Schrittmotors in Kombination mit der Getriebeübersetzung eine ausreichende Selbsthemmung erreicht wird, um ein unbeabsichtigtes Verschieben der Schaftelemente relativ zueinander zu verhindern. Bei Ausführungsformen der Erfindung kann jedoch auch eine Bremse oder Raste vorgesehen sein, insbesondere in Verbindung mit einem einfachen Gleichstrommotor. Eine Bremse bietet den Vorteil einer zuverlässigen Fixierung. Ein ungebremstes System bietet den Vorteil eines einfachen und kompakten Aufbaus und eines kontrollierten Vor- und Rücklaufs.

Bei bevorzugten Ausführungsformen umfasst das zweite Schaftelement eine Zahnstange. Die Zahnstange ist vorzugsweise integral mit dem zweiten Schaftelement ausgeführt oder bildet das zweite Schaftelement. Die Zahnstange wird vorzugsweise von einem Schneckenrad angetrieben. Die Zahnstange bietet den Vorteil ein lediglich durch die Länge des ersten Schaftelements begrenzter Hub ermöglicht wird.

Vorzugsweise weist zumindest eines der Schaftelemente eine Krümmung von höchstens 300 mm Radius auf. Besonders bevorzugt beträgt die Krümmung maximal 220 mm Radius oder 70 mm Radius. Vorteilhafterweise ist auch die Zahnstange gekrümmt, und zwar zumindest im Wesentlichen im gleichen Radius wie zumindest eines der Schaftelemente. Besonders bevorzugt sind beide Schaftelemente und die Zahnstange zumindest im Wesentlichen mit einer identischen Krümmung versehen. Weitere Ausführungsformen der Erfindung weisen zumindest abschnittsweise eine gerade Zahnstange oder eine gerade Spindel für einen Eingriff mit einem Abtriebselement, wie einer Schnecke oder einem Ritzel des Getriebes auf.

Bevorzugte Ausführungsformen der Erfindung weisen ein abtriebsseitig des Getriebes angeordnetes Ritzel auf, welches mit einem Zahnrad in Eingriff steht. Das Zahnrad weist ein Innengewinde auf, welches mit einer Spindel in Eingriff ist. Die Spindel ist vorzugsweise Teil des zweiten Schaftelementes oder bildet das zweite Schaftelement. Bei typischen Ausführungsformen der Erfindung können zusätzliche Stirnräder zwischengeschaltet werden, um eine Baubreite der Vorrichtung zu verringern.

Die Zahnstange oder die Spindel ist vorzugsweise mit dem zweiten Schaftelement verbunden und bewegt sich bei einer Verschiebung der beiden Schaftelemente zueinander innerhalb des ersten Schaftelements. Zum Eingriff eines Antriebszahnrads weist das erste Schaftelement vorzugsweise eine Öffnung auf, besonders bevorzugt eine seitliche Öffnung auf der Längsseite, auf welcher der Elektromotor angeordnet ist. Die Krümmung der Schaftelemente bietet den Vorteil, dass die Skoliosekorrektur anatomisch vorteilhaft ausgeführt werden kann. Durch die seitliche Öffnung wird erreicht, dass die Zahnstange geschützt innerhalb des ersten Schaftelements angeordnet ist, wobei die seitliche Öffnung in dem ersten Schaftelement vorzugsweise durch das Gehäuse des Antriebs umfassend den Elektromotor und das Getriebe bei aufgeschobenem Antrieb verschlossen wird.

Vorzugsweise ist abtriebsseitig des Getriebes eine Schnecke angeordnet, welche die Zahnstange kämmt. Die Schnecke weist vorzugsweise eine Spindelsteigerung von mehr als 0,3 mm, bevorzugter mehr als 0,5 mm oder weniger als 1,5 mm, bevorzugter weniger als 1 mm auf. Vorzugsweise ist die Schnecke, welche durch den Elektromotor, gegebenenfalls mit zwischengeschaltetem Getriebe angetrieben wird, auf einer der ungekrümmten Oberflächen der Zahnstange mit der Zahnstange in Eingriff. Vorteilhafterweise ist die Zahnstange in genau eine Richtung ihres rechteckförmigen Querschnitts gekrümmt. Bei typischen Ausführungsformen sind rechteckförmige Schaftelemente in genau einer Richtung oder einer Ebene gekrümmt. Bei der Zahnstange entstehen so zwei Oberflächen, welche nicht gekrümmt, bzw. lediglich in der Ebene ihrer Oberfläche "um die Kurve" verlaufen. Eine solche Kurve der ebenen Oberfläche ist für einen Eingriff einer Schnecke in die Zähne der Zahnstange unproblematisch.

Vorzugsweise umfasst die Vorrichtung eine mit dem Elektromotor verbundene Kontrolleinrichtung, welche geeignet ist, Energie oder Steuersignale drahtlos zu empfangen. Die Kontrolleinrichtung umfasst vorzugsweise eine Empfangseinheit und elektronische Bauelemente. Die Kontrolleinrichtung ist vorzugsweise implantierbar, insbesondere subkutan anordnebar, und zum dauerhaften Verbleib im menschlichen Körper während einer Skoliosebehandlung bestimmt und geeignet. Auf diese Weise ist es möglich, den Antrieb anzusteuern und mit Energie zu versorgen, ohne dass eine weitere Operation notwendig ist. Die Energieübertragung erfolgt vorzugsweise induktiv. Hierzu umfasst die Kontrolleinrichtung vorzugsweise eine elektromagnetische Spule (Magnetspule). Bevorzugte Ausführungsformen der Kontrolleinrichtung sind energiespeicherlos. Dies bietet den Vorteil eines einfachen Aufbaus. Es hat sich herausgestellt, dass unter Umständen dafür auf einen Energiespeicher verzichtet werden kann. Typische Ausführungsformen der Erfindung weisen hingegen Energiespeicher auf, um die Intensität der elektromagnetischen Strahlung bei Betrieb der Vorrichtung zu verringern.

Zwischen dem Elektromotor und der Kontrolleinrichtung besteht vorzugsweise eine Zuleitung oder Kabelverbindung mit einer elektrisch leitenden Verbindung oder einem elektrisch leitenden Kabel. Die Kontrolleinrichtung ist vorzugsweise dazu eingerichtet, den Antrieb zu steuern oder zu regeln und umfasst vorzugsweise einen integrierten Schaltkreis, um entsprechende Funktionen durchzuführen. Dies bietet den Vorteil eines kompakten Aufbaus bei großer Funktionalität.

Bevorzugte Ausführungsformen der Kontrolleinrichtung umfassen einen Rückkanal, sodass es ermöglicht wird, Informationen von der Vorrichtung oder der Kontrolleinrichtung, wenn diese implantiert sind, an ein externes Steuergerät zu übertragen. Vorzugsweise ist die Kontrolleinrichtung für eine bidirektionale Datenübertragung eingerichtet.

Vorzugsweise ist die Kontrolleinrichtung dazu eingerichtet, den Antrieb so anzusteuern, dass ein Auseinanderfahren und ein Ineinanderfahren der Schaftelemente möglich ist. Dies bietet den Vorteil, dass auch ein Zurückfahren möglich ist, beispielsweise falls eine zu große Kraft festgestellt wird. Vorzugsweise sind ebenso der Elektromotor, das Getriebe oder andere Teile der Vorrichtung entsprechend ausgebildet, um ein Vorwärts- und Rückwärtsfahren der beiden Schaftelemente relativ zueinander zu ermöglichen.

Vorzugsweise umfasst die Vorrichtung einen Sensor, der angeordnet ist, um eine relative Verschiebung oder eine Kraft zwischen den Schaftelementen zu erfassen. Vorzugsweise ist die Kontrolleinrichtung dazu geeignet, Informationen des Sensors zu verarbeiten oder zu übertragen, vorzugsweise an ein außerhalb des Körpers angeordnetes Steuergerät. Weiterhin umfasst die Kontrolleinrichtung vorzugsweise eine Regeleinheit, welche anhand des Sensorsignals den Elektromotor ansteuert, um einen vorgegebenen Kraftwert oder Verschiebungswert zu erreichen. Ebenso ist es möglich, die Regelschleife über ein externes Steuergerät aufzubauen. Dies bietet den Vorteil einer exakten Einstellung.

Vorzugsweise umfasst das Steuergerät, welches außerhalb des Körpers angeordnet wird, eine elektromagnetische Spule, um Energie und Signale an die Kontrolleinrichtung induktiv zu übertragen. Das Steuergerät ist vorzugsweise eingerichtet, um die Vorrichtung drahtlos über die Kontrolleinrichtung zu steuern.

### Kurze Beschreibung der Zeichnungen

Bevorzugte Ausführungsbeispiele der Erfindung werden anhand der beiliegenden Zeichnungen erläutert, wobei die Figuren zeigen:
- Fig. 1: zeigt in einer schematischen, teilweise geschnittenen Draufsicht eine erfindungsgemäße Vorrichtung;
- Fig. 2: zeigt in einer perspektivischen Ansicht einen Teil der erfindungsgemäßen Vorrichtung der Fig. 1;
- Fig. 3: zeigt in einer schematischen, teilweise geschnittenen Ansicht einen Teil der Vorrichtung der Fig. 1; und
- Fig. 4: zeigt in einer schematischen, teilweise geschnittenen Ansicht eine weitere erfindungsgemäße Ausführungsform einer Vorrichtung zur Skoliosebehandlung zusammen mit einem Steuergerät als System zur Skoliosebehandlung.

### Beschreibung bevorzugter Ausführungsbeispiele

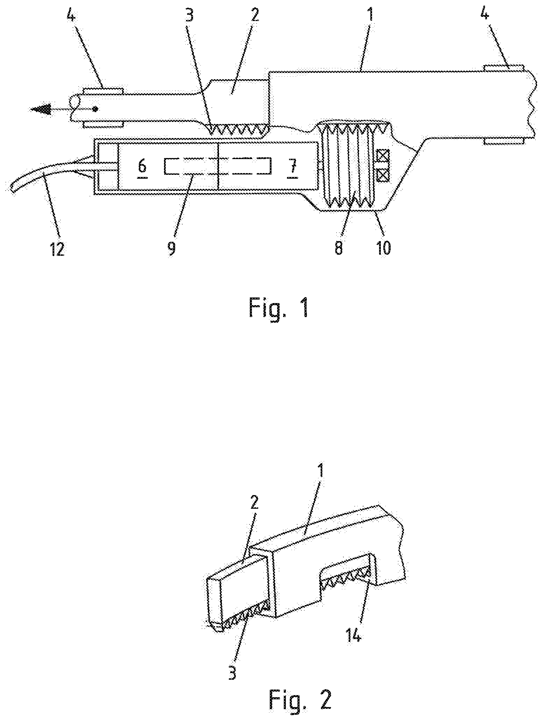

In der Fig. 1 ist schematisch in einer teilweise geschnittenen Ansicht eine bevorzugte Ausführungsform einer erfindungsgemäßen Vorrichtung gezeigt.

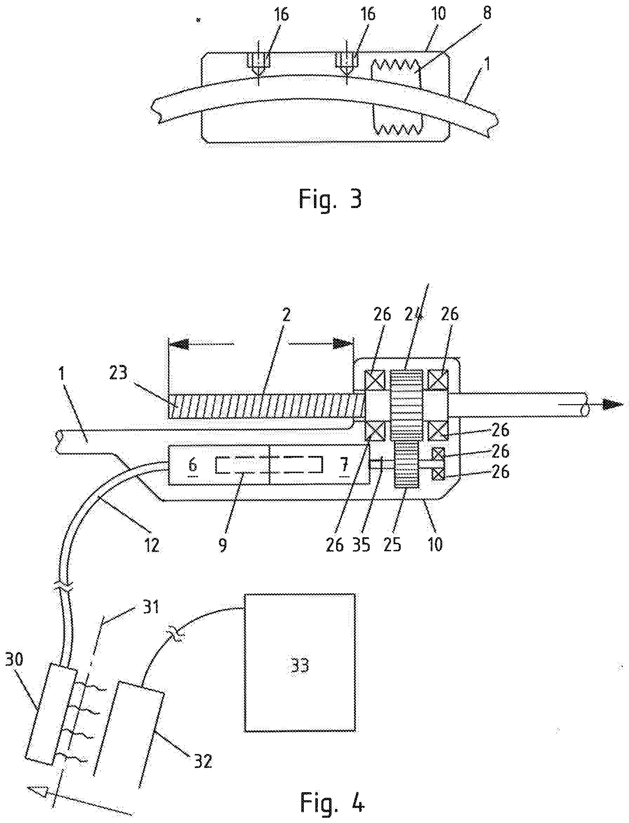

Die erfindungsgemäße Vorrichtung der Fig. 1 umfasst ein erstes Schaftelement 1 und ein zweites Schaftelement 2. Das zweite Schaftelement 2 umfasst integral eine Zahnstange 3. Die Zahnstange 3 bildet das zweite Schaftelement 2. An den jeweiligen Enden der Schaftelemente 1 und 2 sind Knochenbefestigungen 4 vorgesehen, welche dazu geeignet sind, in Rippenbögen einzugreifen. Das zweite Schaftelement 2 ist in Richtung eines Pfeils, welcher in der Fig. 1 gezeigt wird, relativ zu dem ersten Schaftelement 1 in beiden Richtungen, d.h. bidirektional verschiebbar. Um eine solche Verschiebung zu erreichen, wird ein Elektromotor 6 verwendet, welcher über ein Planetenradgetriebe 7 mit einer Übersetzung ein Schneckenrad 8 antreibt. Das Schneckenrad 8 wird über eine Abtriebswelle 9 (strichliert dargestellt) des Elektromotors 6 und das Planetenradgetriebe 7 angetrieben. Der Elektromotor 6, das Planetenradgetriebe 7 und das Schneckenrad 8 sind in einem Gehäuse 10 angeordnet, welches seitlich auf das erste Schaftelement 1 aufgeschoben ist und auf dem ersten Schaftelement 1 fixiert ist. Die Befestigung des Gehäuses 10 auf dem ersten Schaftelement 1 ist in der Fig. 3 genauer dargestellt.

Die in der Fig. 1 gezeigte Vorrichtung kann vorteilhafterweise zur Skoliosebehandlung eingesetzt werden, wobei die beiden Knochenbefestigungen 4 mit Rippenbögen oder Wirbelkörpern verbunden werden. Durch ein Auseinandertreiben der Schaftelemente 1 und 2 wird erreicht, dass eine Wirbelsäule aufgerichtet wird. Das Auseinanderfahren der Schaftelemente 1 und 2 kann dabei über einen langen Zeitraum schrittweise ohne operative Zwischeneingriffe vorgenommen werden.

Der Vorteil der dargestellten Vorrichtung ist, dass sie vollständig implantierbar ist. Der Elektromotor 6 ist über eine Zuleitung 12 oder ein Kabel mit einer ebenfalls zur Implantation vorgesehen Kontrolleinrichtung (in der Fig. 1 nicht gezeigt) verbunden. Die Kontrolleinrichtung umfasst eine Elektronik und einen Empfänger, verbleibt ebenfalls im Körper und ist geeignet, Steuersignale und Energie zur Ansteuerung und zur Energieversorgung des Elektromotors 6 induktiv zu empfangen und auszuwerten. Dies wird eingehender im Zusammenhang mit dem Ausführungsbeispiel der Fig. 4 erläutert.

Durch die hohe Übersetzung des Planetenradgetriebes 7 und die Schneckenrad-Zahnstangenübertragung zwischen der Zahnstange 3 und dem Schneckenrad 8 wird eine große Selbsthemmung zwischen dem Elektromotor 6 und dem angetriebenen zweiten Schaftelement 2 erreicht, sodass eine Bremse oder eine Raste nicht zwingend notwendig sind, um ohne Energiezufuhr ein Halten der Schaftelemente 1 und 2 in einer einmal angefahrenen Position sicherzustellen. Auf diese Weise wird ein sehr einfaches System geschaffen, welches zusätzlich ein Vor- und Zurückfahren des zweiten Schaftelementes 2 relativ zu dem ersten Schaftelement 1 ermöglicht.

Die Schaftelemente 1 und 2 der in der Fig. 1 dargestellten Vorrichtung sind gekrümmt, wobei die Krümmungsebene senkrecht zur Zeichenebene steht. Dies bedeutet, dass die Oberfläche der Zahnstange 3, auf welcher die Zähne der Zahnstange 3 angeordnet sind, ungekrümmt bzw. eben ist. Dabei bedeutet "eben" wiederum mit Ausnahme der Erhebungen der Zähne der Zahnstange 3. Der Vorteil der in der Fig. 1 gezeigten Anordnung mit der beschriebenen, beispielhaften Krümmung ist, dass die Krümmung von 220 mm Radius vorteilhafterweise für eine Skoliosebehandlung eingesetzt werden kann. Andere bevorzugte Ausführungsformen weisen eine Krümmung von 70 mm Radius auf. Gleichzeitig ist eine Anordnung des Elektromotors auf der gezeigten Längsseite der Schaftelemente 1 und 2 vorteilhaft, da gerade an dieser Stelle aus medizinischer Sicht Platz ist.

In der Fig. 2 sind noch einmal Ausschnitte der Schaftelemente 1 und 2 der Fig. 1 dargestellt. Bei der Beschreibung der Figuren 2 bis 4 werden gleiche Bezugszeichen für gleiche oder ähnliche Teile verwendet.

In der Fig. 2 ist die Krümmung der Schaftelemente 1 und 2 zu erkennen, wobei das rechteckförmige, massive zweite Schaftelement 2 in dem rechteckförmigen Rohr des ersten Schaftelements 1 geführt ist. Weiterhin ist in der Fig. 2 eine Öffnung 14 dargestellt, welche für den Eingriff des Schneckenrads 8 in die Zahnstange 3 vorgesehen ist.

Bei typischen Ausführungsformen ist das zweite Schaftelement ebenfalls hohl oder rohrförmig ausgeführt, so kann das zweite Schaftelement als hohle Zahnstange oder Spindel ausgeführt werden. Dies bieten den Vorteil eines kompakten und leichten Aufbaus. Bei weiteren typischen Ausführungsformen erfolgt die Kraftübertragung über ein Ritzel oder ein Gewinde.

In der Fig. 3 ist gezeigt, wie das Gehäuse 10 auf das Schaftelement 1 aufgeschoben ist. Die Schnittansicht zeigt, wie das Gehäuse 10 mit zwei Schrauben 16 an dem Schaftelement 1 fixiert werden kann. In der Fig. 1 ist das Schaftelement 1 teilweise geschnitten dargestellt, wie auch das Gehäuse 10 in der Fig. 1 teilweise geschnitten dargestellt ist, um das Schneckenrad 8, den Motor 6 und das Getriebe 7 darzustellen.

In der Fig. 4 ist eine weitere erfindungsgemäße Ausführungsform der Erfindung gezeigt, wobei in der Fig. 4 insbesondere auch die Kontrolleinrichtung 30 gezeigt ist, welche ebenso bei der Vorrichtung der Fig. 1 vorteilhafterweise vorgesehen ist. Wiederum werden für gleiche oder ähnliche Teile gleiche Bezugszeichen verwendet.

Die Ausführungsform der Fig. 4 weist zunächst zwei Unterschiede zu der Ausführungsform der Fig. 1 auf. Der Elektromotor 6 und das Getriebe 7 sind seitlich des Schaftelementes 2 angeordnet, jedoch ist das Gehäuse 10, in welchem der Elektromotor 6 und das Planetenradgetriebe 7 angeordnet sind, integriert mit dem ersten Schaftelement 1 ausgebildet. Eine Kraftübertragung von der Abtriebswelle 9 auf das Schaftelement 2 ist über eine Spindel 23, welche Teil des Schaftelements 2 ist, möglich. Die Spindel 23 steht mit einem Innengewinde eines Zahnrades 24 in Eingriff, welches durch ein Ritzel 25 angetrieben wird. Das Ritzel 25 wird über das Planetenradgetriebe 7 durch den Elektromotor angetrieben. Zur Lagerung des Schaftelements 2 an dem integral mit dem Schaftelement 1 ausgebildeten Gehäuse 10 und zur Lagerung des Ritzels sind Lager 26 vorgesehen.

Der Elektromotor 6 ist über die Zuleitung 12 mit der Kontrolleinrichtung 30 verbunden, welche induktiv mit Energie und Steuersignalen versorgt wird. Die Kontrolleinrichtung 30 ist dazu vorgesehen, im menschlichen Körper implantiert zu werden, insbesondere unter einer Haut 31 des menschlichen Körpers. Zur Energie- und Steuersignal-Aussendung und zum Empfang von Rückkanaldaten, d.h. für einen bidirektionalen Datenaustausch wird eine Übertragungseinheit 32 verwendet, welche mit einer Steuereinheit 33 verbunden ist. Die Steuereinheit 33 und die Übertragungseinheit 32 bilden zusammen ein Steuergerät, welches geeignet ist, die Vorrichtung, welche die innerhalb des Körpers angeordneten Elemente der Erfindung umfasst, drahtlos über die Kontrolleinrichtung 30 zu steuern und induktiv mit Energie zu versorgen. So ist es mit der Steuereinheit 33 möglich, über die Kontrolleinrichtung 30 den Elektromotor 6, welcher als Schrittmotor ausgebildet ist, derart anzusteuern, dass ein bestimmter Hub der Schaftelemente 1 und 2 relativ zueinander erreicht wird.

Über einen Sensor 35, welcher an der Planetenradgetriebe-Abtriebswelle angeordnet ist, kann eine Momentenbelastung dieser Welle überprüft werden und auf diese Weise auf die Kraft rückgeschlossen werden, welche zwischen den Schaftelementen 1 und 2 vorherrscht. Diese Kraft wird durch die Kontrolleinrichtung 30 über einen Rückkanal an die Übertragungseinheit 32 und weiter an die Steuereinheit 33 übertragen. Die Steuereinheit 33 kann bei einer zu großen Kraft einen Vorschub des Elektromotors 6 durch die oben beschriebene Ansteuerung über die Kontrolleinrichtung 30 stoppen. Auf diese Weise lässt sich ein Regelkreis einrichten.

Bei vorteilhaften Ausführungsformen der Erfindung ist unter einem der Lager 26 ein Sensor angeordnet, welcher ebenfalls verwendet werden kann, um die Kraft, welche zwischen den Schaftelementen herrscht zu bestimmen. Dies bietet den Vorteil eines einfachen Aufbaus.

Bei typischen Ausführungsformen der Erfindung ist das Gehäuse, in welchem der Elektromotor und das Planetenradgetriebe angeordnet sind, integral mit dem ersten Schaftelement ausgebildet. Dies bietet den Vorteil eines einfachen Aufbaus. Der Elektromotor ist dann längsseits benachbart zu dem zweiten Schaftelement angeordnet. Dabei bedeutet "längsseits benachbart" in dieser Anmeldung allgemein, dass der Elektromotor längsseits benachbart zu einer Achse oder einer Ausdehnung des ersten oder zweiten Schaftelements angeordnet ist, auch wenn im Betrieb das entsprechende Schaftelement nicht mehr exakt seitlich neben dem Elektromotor ist, sondern aufgrund eines Herausfahrens oder Auseinanderfahrens der Vorrichtung verschoben ist.

Bei weiteren Ausführungsformen der Erfindung kann der Sensor auch eine Wegstrecke bzw. eine Umdrehung messen und diese mittels des Rückkanals über die Kontrolleinrichtung an das Steuergerät übertragen, um so, falls ein einfacher Gleichstrommotor ohne Schrittmotoreigenschaften verwendet wird, einen Regelkreis aufzubauen, um bestimmte Hublängen der Schaftelemente anzusteuern.

Die Erfindung ist nicht auf die vorstehend beschriebenen bevorzugten Ausführungsformen beschränkt. Der Umfang der Erfindung wird vielmehr durch die Ansprüche bestimmt.

## Patentansprüche

1. Skoliosebehandlungsvorrichtung zur relativen Verschiebung von Knochen zur Behandlung von Skoliose beim Menschen, mit:
- einem ersten Schaftelement (1),
- einem zweiten Schaftelement (2), das verschiebbar mit dem ersten Schaftelement (1) verbunden ist,
- einem Elektromotor, welcher mit dem ersten Schaftelement (1) verbunden ist, wobei eine Abtriebswelle des Elektromotors mit dem zweiten Schaftelement (2) in Wirkverbindung steht, um eine Verschiebung der Schaftelemente (1, 2) relativ zueinander zu ermöglichen, und
- eine mit dem Elektromotor (6) verbundene Kontrolleinrichtung (30), welche geeignet ist, Energie drahtlos zu empfangen, um den Elektromotor (6) mit Energie zu versorgen,
- wobei zumindest eines der Schaftelemente (1, 2) eine Knochenbefestigung (4) aufweist, welche geeignet ist, mit Rippenbögen oder Wirbelkörpern verbunden zu werden
**dadurch gekennzeichnet, dass**
- der Elektromotor (6) längsseits benachbart von zumindest einem der Schaftelemente (1, 2) angeordnet ist.

2. Skoliosebehandlungsvorrichtung nach Anspruch 1, **gekennzeichnet durch** ein Getriebe, welches in der Wirkverbindung zwischen der Abtriebswelle und dem zweiten Schaftelement (2) angeordnet ist.

3. Skoliosebehandlungsvorrichtung nach Anspruch 2, **dadurch gekennzeichnet, dass** das Getriebe eine Übersetzung von mindestens 1:250 aufweist.

4. Skoliosebehandlungsvorrichtung nach Anspruch 1 oder 2, **dadurch gekennzeichnet, dass** das Getriebe ein Planetengetriebe (7) umfasst.

5. Skoliosebehandlungsvorrichtung nach einem der vorhergehenden Ansprüche, **dadurch gekennzeichnet, dass** der Elektromotor in einem Gehäuse angeordnet ist, welches seitlich auf eine Längsseite des ersten Schaftelementes (1) lösbar aufgeschoben ist.

6. Skoliosebehandlungsvorrichtung nach einem der vorhergehenden Ansprüche, **dadurch gekennzeichnet, dass** die Schaftelemente (1, 2) teleskopartig ineinander verschiebbar sind.

7. Skoliosebehandlungsvorrichtung nach einem der vorhergehenden Ansprüche, **dadurch gekennzeichnet, dass** zumindest eines der Schaftelemente (1, 2) eine Krümmung von höchstens 300mm Radius aufweist.

8. Skoliosebehandlungsvorrichtung nach einem der vorhergehenden Ansprüche, **dadurch gekennzeichnet, dass** die Kontrolleinrichtung (30) geeignet ist, Steuersignale drahtlos zu empfangen.

9. Skoliosebehandlungsvorrichtung nach Anspruch 8, **dadurch gekennzeichnet, dass** die Kontrolleinrichtung (30) dazu eingerichtet ist, den Elektromotor (6) so anzusteuern, dass ein Auseinanderfahren und ein Ineinanderfahren der Schaftelemente (1, 2) möglich ist.

10. Skoliosebehandlungsvorrichtung nach einem der vorhergehenden Ansprüche, **gekennzeichnet durch** einen Sensor (35), der angeordnet ist, um eine relative Verschiebung und/oder eine Kraft zwischen den Schaftelementen (1, 2) zu erfassen.

11. Skoliosebehandlungsvorrichtung nach einem der vorhergehenden Ansprüche, **dadurch gekennzeichnet, dass** das zweite Schaftelement (2) eine Zahnstange (3) umfasst, welche mit einem durch den Elektromotor (6) angetriebenen Schneckenrad (8) in Eingriff steht.

12. Skoliosebehandlungsvorrichtung nach Anspruch 11, **dadurch gekennzeichnet, dass** das Schneckenrad (8) auf einer ungekrümmten Oberfläche der Zahnstange (3) in Zähne der Zahnstange (3) eingreift.

13. Skoliosebehandlungsvorrichtung nach einem der Ansprüche 1 bis 10, **dadurch gekennzeichnet, dass** das zweite Schaftelement (2) eine Spindel (23) umfasst, welche mit einem durch den Elektromotor (6) angetriebenen Zahnrad (24) mit Innengewinde in Eingriff steht.

14. System zur Skoliosekorrektur mit einer Skoliosebehandlungsvorrichtung nach einem der vorhergehenden Ansprüche zur Implantation in einen menschlichen Körper, **gekennzeichnet durch** ein Steuergerät (32, 33), welches dazu eingerichtet ist, die Skoliosebehandlungsvorrichtung drahtlos über die Kontrolleinrichtung (30) zu steuern und induktiv mit Energie zu versorgen.

## Claims

1. Scoliosis treatment device for the relative movement of bones for the treatment of scoliosis in humans, with:
- a first shaft element (1),
- a second shaft element (2), which is connected movably to the first shaft element (1), and
- an electric motor, which is connected to the first shaft element (1), wherein an output shaft of the electric motor is operatively connected to the second shaft element (2) in order to permit a movement of the shaft elements (1, 2) relative to each other, and
- a control means (30), which is connected to the electric motor (6) and is suitable for receiving energy wirelessly to supply the electric motor (6) with energy,
- wherein at least one of the shaft elements (1, 2) comprises a bone fixation means (4), which is suitable for being connected with costal arches or vertebral bodies,
**characterized in that**
- the electric motor (6) is arranged lengthways adjacent to at least one of the shaft elements (1, 2).

2. Scoliosis treatment device according to Claim 1, **characterized by** a gear arranged in the operative connection between the output shaft and the second shaft element (2).

3. Scoliosis treatment device according to Claim 2, **characterized in that** the gear has a transmission ratio of at least 1:250.

4. Scoliosis treatment device according to Claim 1 or 2, **characterized in that** the gear comprises a planetary gear (7).

5. Scoliosis treatment device according to one of the preceding claims, **characterized in that** the electric motor is arranged in a housing, which is pushed laterally and releasably onto a lengthwise side of the first shaft element (1).

6. Scoliosis treatment device according to one of the preceding claims, **characterized in that** the shaft elements (1, 2) are movable telescopically one inside the other.

7. Scoliosis treatment device according to one of the preceding claims, **characterized in that** at least one of the shaft elements (1, 2) has a curvature of at most 300 mm radius.

8. Scoliosis treatment device according to one of the preceding claims, **characterized by** the control means (30) is suitable for receiving control signals wirelessly.

9. Scoliosis treatment device according to Claim 8, **characterized in that** the control means (30) is designed to control the electric motor (6) in such a way that it is possible for the shaft elements (1, 2) to be driven apart and to be driven one inside the other.

10. Scoliosis treatment device according to one of the preceding claims, **characterized by** a sensor (35), which is arranged to detect a relative movement and/or a force between the shaft elements (1, 2).

11. Scoliosis treatment device according to one of the preceding claims, **characterized in that** the second shaft element (2) comprises a toothed rack (3), which is in engagement with a worm wheel (8) driven by the electric motor (6).

12. Scoliosis treatment device according to Claim 11, **characterized in that** the worm wheel (8) engages in teeth of the toothed rack (3) on an uncurved surface of the toothed rack (3).

13. Scoliosis treatment device according to one of Claims 1 through 10, **characterized in that** the second shaft element (2) comprises a spindle (23), which is in engagement with a toothed wheel (24) that is driven by the electric motor (6) and that has an internal thread.

14. System for correcting scoliosis with a scoliosis treatment device according to one of the preceding claims for implantation in a human body, **characterized by** a controller (32, 33) designed to control the scoliosis treatment device wirelessly via the control means (30) and to supply it inductively with energy.

## Revendications

1. Dispositif de traitement de la scoliose pour un déplacement relatif d'os pour le traitement de la scoliose chez l'être humain, avec:
- un premier élément d'arbre (1),
- un deuxième élément d'arbre (2) qui est connecté de manière déplaçable au premier élément d'arbre (1),
- un moteur électrique qui est connecté audit premier élément d'arbre (1), un arbre de sortie du moteur électrique étant connecté en fonctionnement au deuxième élément d'arbre (2) pour permettre un déplacement des éléments d'arbre (1, 2) l'un par rapport à l'autre, et
- un moyen de commande (30) connecté au moteur électrique (6) qui convient pour recevoir sans fil de l'énergie pour alimenter le moteur électrique (6) en énergie,
- dans lequel au moins l'un des éléments d'arbre (1, 2) présente une fixation d'os (4) qui convient pour être connectée à des arcs costaux ou des corps vertébraux,
**caractérisé par le fait que**
- le moteur électrique (6) est disposé longitudinalement adjacent à au moins l'un des éléments d'arbre (1, 2).

2. Dispositif de traitement de la scoliose selon la revendication 1, **caractérisé par** une transmission qui est disposée en liaison active entre l'arbre de sortie et le deuxième élément d'arbre (2).

3. Dispositif de traitement de la scoliose selon la revendication 2, **caractérisé par le fait que** la transmission présente un ratio d'au moins 1: 250.

4. Dispositif de traitement de la scoliose selon la revendication 1 ou 2, **caractérisé par le fait que** la transmission comporte un engrenage planétaire (7).

5. Dispositif de traitement de la scoliose selon l'une des revendications précédentes, **caractérisé par le fait que** le moteur électrique est disposé dans un boîtier qui est engagé de manière amovible latéralement sur un côté longitudinal du premier élément d'arbre (1).

6. Dispositif de traitement de la scoliose selon l'une des revendications précédentes, **caractérisé par le fait que** les éléments d'arbre (1, 2) peuvent se déplacer de manière télescopique l'un dans l'autre.

7. Dispositif de traitement de la scoliose selon l'une des revendications précédentes, **caractérisé par le fait qu'**au moins l'un des éléments d'arbre (1, 2) présente une courbure d'un rayon de tout au plus 300 mm.

8. Dispositif de traitement de la scoliose selon l'une des revendications précédentes, **caractérisé par le fait que** le moyen de commande (30) convient pour recevoir sans fil des signaux de commande.

9. Dispositif de traitement de la scoliose selon la revendication 8, **caractérisé par le fait que** le moyen de commande (30) est conçu pour activer le moteur électrique (6) de sorte que soit possible un déplacement l'un hors de l'autre et l'un dans l'autre des éléments d'arbre (1, 2).

10. Dispositif de traitement de la scoliose selon l'une des revendications précédentes, **caractérisé par** un capteur (35) qui est aménagé pour détecter un déplacement relatif et/ou une force entre les éléments d'arbre (1, 2).

11. Dispositif de traitement de la scoliose selon l'une des revendications précédentes, **caractérisé par le fait que** le deuxième élément d'arbre (2) comporte une crémaillère (3) qui est en prise avec une roue hélicoïdale (8) entraînée par le moteur électrique (6).

12. Dispositif de traitement de la scoliose selon la revendication 11, **caractérisé par le fait que** la roue hélicoïdale (8) vient en prise, sur une surface non-courbée de la crémaillère (3), avec des dents de la crémaillère (3).

13. Dispositif de traitement de la scoliose selon l'une des revendications 1 à 10, **caractérisé par le fait que** le deuxième élément d'arbre (2) comporte une broche (23) qui est en prise avec une roue dentée (24) taraudée entraînée par le moteur électrique (6).

14. Système pour la correction de la scoliose à l'aide d'un dispositif de traitement de la scoliose selon l'une des revendications précédentes pour l'implantation dans un corps humain, **caractérisé par** un dispositif de commande (32, 33) qui est conçu pour commander le moyen de traitement de la scoliose sans fil par le moyen de commande (30) et pour l'alimenter en énergie de manière inductive.
